# DEMANDE DE BREVET EUROPEEN

(11) **EP 0 646 364 A1**
(43) Date de publication de la demande: **05.04.1995**
(21) Numéro de dépôt: 94402192.2
(22) Date de dépôt: 30.09.1994
(51) Int. Cl.: A61F 2/02

(54) **Dispositif d'implantation d'une prothèse médicale dans un conduit d'un corps humain ou animal**

(30) Priorité: 05.10.1993 FR 9311851
(71) Demandeur: B. BRAUN CELSA, Société Anonyme, F-86360 Chasseneuil du Poitou (FR)
(72) Inventeur: Roussigne, Maurice, F-86000 Poitiers (FR); Nadal, Guy, F-86000 Poitiers (FR); Chevillon, Gérard, F-92120 Montrouge (FR)
(74) Mandataire: Lerner, François

(57) **Abrégé**

Il s'agit d'un dispositif d'implantation d'une prothèse médicale (1) dans un conduit d'un corps humain ou animal.

Ce dispositif comprend une gaine tubulaire (8), adaptée pour contenir la prothèse à implanter et une tige (24) de commande de la prothèse, pouvant glisser à l'intérieur de la gaine. La gaine et/ou la tige est (sont) pourvue(s) sur une partie au moins de sa (leur) longueur de moyens flexibles de centrage (54) propres à agir sur le profil de la ligne axiale de la gaine, en contraignant son extrémité distale (82) à s'aligner sensiblement avec l'axe du conduit (2), lorsque la gaine y est disposée.

L'invention trouve tout particulièrement son application dans le domaine des filtres sanguins implantables dans un vaisseau sanguin.

## Description

L'invention a en particulier pour objet un dispositif perfectionné d'implantation d'une prothèse dans un conduit interne d'un corps humain ou animal vivant.

L'invention trouve son application dans le domaine médical, lorsqu'il est nécessaire d'implanter une prothèse dans une zone déterminée d'un conduit du corps du patient, telle en particulier un filtre sanguin implanté pour retenir des caillots de sang.

Des dispositifs de ce type sont par exemple décrits dans la demande FR-A-2 657 261. Le dispositif comprend en général une gaine ordinairement souple qui s'adapte aisément à des trajets sinueux. Cette gaine est facilement insérée dans le conduit grâce à un fil de guidage et à un tube-introducteur. Ce dispositif comprend également une tige de commande, propre à coulisser dans la gaine, notamment pour expulser la prothèse hors de cette gaine.

Les contrôles généralement effectués après l'implantation ont montré que parfois la prothèse n'était pas convenablement positionnée dans le conduit.

Ces problèmes ont été tout particulièrement observés pour les filtres sanguins.

Parmi ces filtres on en connait qui se présentent en position déployée sous la forme d'un panier tronconique à pattes élastiques, et qui peuvent être repliés sur eux-mêmes pour leur implantation. De tels filtres sont souvent mis en place dans la veine cave inférieure, par voie d'accès percutanée ou par "dénudation", soit au niveau de la veine jugulaire (via la veine cave supérieure) soit au niveau d'une des veines fémorales. C'est en particulier dans le cas d'une introduction par voie fémorale qu'il a été constaté une ouverture parfois dissymétrique des filtres dans les vaisseaux, alors même qu'il s'agissait de filtres dits "autocentrants", c'est-à-dire censés se déployer naturellement avec leur axe longitudinal sensiblement confondu avec celui du vaisseau. Cette ouverture incorrecte est tout spécialement préjudiciable pour les filtres "définitifs" ou "permanents" qui sont munis de moyens d'accrochage à la paroi du vaisseau et dont la position ne peut pratiquement pas être corrigée après ouverture.

Face à ces problèmes, la demanderesse, au lieu de chercher à perfectionner encore la capacité d'autocentrage des filtres expansibles ou, plus généralement, les propriétés de centrage des prothèses, s'est au contraire intéressée au dispositif utilisé pour leur implantation, et a pu constater que notamment la gaine extérieure, nécessaire à l'implantation, se trouvait parfois plaquée contre la paroi du conduit, du moins dans sa partie terminale. Cela a notamment été constaté lors d'implantations de filtres sanguins par voie fémorale, là où les veines iliaques gauche et droite forment un coude avec la veine cave, définissant ainsi un trajet veineux courbe sensiblement en forme de "S".

Il a été supposé que ceci pouvait expliquer que la prothèse, une fois éjectée hors de la gaine, puisse se positionner de travers par rapport à l'axe du conduit, avec les risques inhérents que l'on imagine (traumatismes de la paroi du vaisseau, efficacité moindre de la prothèse, risques accrus de migration...).

C'est dans ces conditions que l'invention propose un dispositif d'implantation comprenant :
- une gaine tubulaire pouvant contenir au moins localement une prothèse à implanter, la gaine qui est allongée suivant une ligne axiale présente une extrémité distale et est flexible sur une partie au moins de sa longueur,
- une tige de commande de la prothèse, au moins en partie flexible et adaptée pour glisser dans la gaine ; ce dispositif se caractérisant en ce que la gaine et/ou la tige est (sont) pourvue(s) ou bien équipée(s) sur une partie au moins de sa (leur) longueur de moyens flexibles de centrage propres à agir sur le profil de la ligne axiale de la gaine, en contraignant son extrémité distale à s'orienter vers l'axe du conduit lorsque la gaine y est disposée.

A toutes fins utiles, on notera que l'expression "extrémité distale" désignera l'extrémité implantée le plus profondément dans le conduit, l'"extrémité proximale" étant bien entendu l'extrémité opposée devant être située en dehors du corps du patient.

Avantageusement, les moyens de centrage prévus dans l'invention vont contraindre, par réaction et à distance, l'orientation en particulier de l'extrémité distale de la gaine, cette dernière étant, une fois implantée, soumise en fait aux contraintes exercées sur elle à la fois par les moyens de centrage et par son environnement.

L'invention et sa mise en oeuvre apparaîtront plus clairement à l'aide de la description qui va suivre faite en référence aux dessins annexés, dans lesquels :
- la figure 1 est une vue en perspective montrant l'implantation d'une prothèse médicale de type connu ;
- les figures 2 à 5 montrent les principaux éléments d'un dispositif utilisé classiquement pour la mise en place de la prothèse de la figure 1 ;
- la figure 6 est une vue schématique agrandie en coupe du détail repéré en VI sur la figure 5 ;
- la figure 7 illustre schématiquement, en partie en coupe, la position de la gaine pour l'implantation selon l'art antérieur de la prothèse de la figure 1 ;
- la figure 8 représente la position rectifiée de la gaine de la figure 7 en place dans son conduit ;
- la figure 9 représente, à plus grande échelle, un mode de réalisation du câble de centrage du dispositif selon l'invention, utilisé conjointement avec les autres éléments illustrés aux figures 2 à 6 ;
- la figure 10 est une vue en coupe du détail repéré X sur la figure 8 ;
- la figure 11 montre, avec la figure 10, deux étapes successives de la mise en place d'une prothèse médicale grâce au dispositif selon l'invention ;
- la figure 12 montre la forme adoptée à la température ambiante par un câble selon l'invention réalisé en un matériau à mémoire de forme thermique ;
- la figure 13 représente le câble de la figure 12 à une température plus élevée ;
- la figure 14 représente une variante de réalisation de la gaine et de la tige de commande ; et
- la figure 15 est une vue agrandie en coupe de l'extrémité distale de la tige de la figure 14.

On ne décrira ci-après l'utilisation du dispositif selon l'invention que pour l'implantation d'un filtre sanguin "définitif". Il est toutefois clair que l'invention peut s'appliquer à l'implantation d'autres filtres, voire d'autres prothèses (par exemple, des "stents"), même non vasculaires (prothèses prostatiques, par exemple).

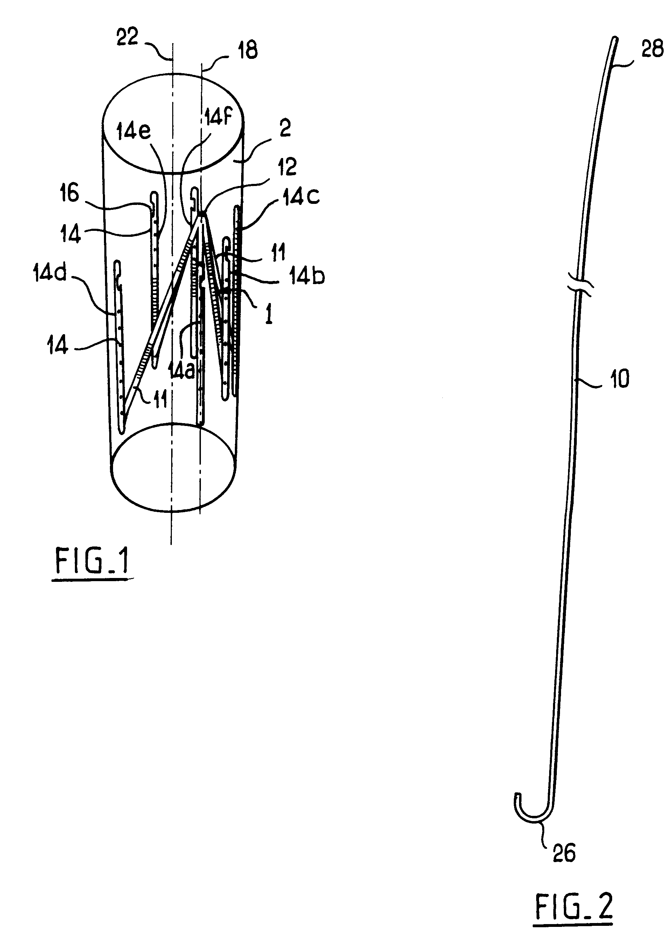

A la figure 1, un filtre sanguin 1, connu et par exemple décrit dans le brevet FR-A-2 573 646, est implanté dans une veine cave inférieure 2. Ce filtre auto-centrant et définitif est formé de six pattes élastiques 11, issues d'une tête commune 12 et déployables (ou auto-expansibles) radialement suivant une corolle conique. Ces pattes sont munies d'appendices de centrage 14 pour favoriser le centrage du filtre dans l'axe 22 du vaisseau. Chaque appendice comporte un crochet 16 de fixation du filtre à la paroi du vaisseau 2. Le filtre est ici incorrectement positionné dans le conduit 2, son axe longitudinal 18 étant décalé par rapport à l'axe 22.

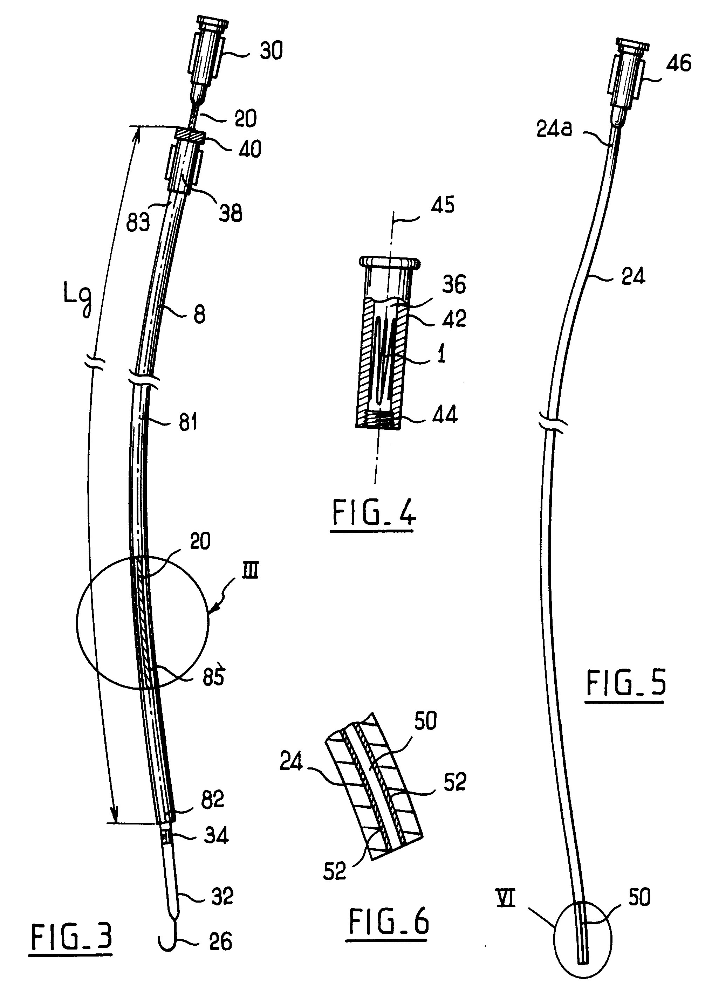

Aux figures 2 à 6, on voit représenté les éléments d'un dispositif connu de mise en place du filtre, à savoir un fil de guidage 10, un mandrin 20, une gaine 8 et une tige de commande 24. Ces éléments, exception faite du fil de guidage qui sera de préférence métallique, seront a priori réalisés en matière plastique biocompatible, par exemple en polyoléfine. Ils seront suffisamment souples ou flexibles pour pouvoir glisser le long de la voie d'accès sans risque d'abîmer les tissus.

Le fin fil de guidage 10, à extrémité distale en "J" 26, peut avoir un diamètre de l'ordre de 0,5 à 1 mm). Le mandrin tubulaire 20 adapté pour pouvoir coulisser sur le fil 10 comprend une tête tubulaire proximale élargie 30 et, vers son extrémité distale 32, une bague métallique radio-opaque 34.

Le diamètre intérieur de la gaine tubulaire 8 est adapté pour que le mandrin 20, ou la tige 24, puisse y coulisser avec un léger jeu radial, et que le filtre, en position rétractée, puisse y glisser sans jeu radial important. Cette gaine, de préférence à rigidité axialement constante, a une longueur L_{g}, suivant sa ligne axiale 81 et est adaptée pour que le repère 34 ressorte juste à son extrémité distale 82, lorsque le mandrin est totalement enfoncé dans son passage intérieur 85. A son extrémité proximale 83, elle comprend un embout creux élargi 38 fileté en 40.

En outre, le dispositif comprend un corps de seringue 42 en plastique, propre à recevoir le filtre 1 rétracté.

La tige de commande 24, dont la longueur est en pratique légèrement supérieure à celle de la gaine, se termine à son extrémité proximale 24a par un embout creux élargi 46 pouvant s'emboîter dans celui 38 de la gaine. Avantageusement, la tige 24 sera creuse pour le coulissement à travers son passage intérieur 50 d'un câble de centrage 54 décrit ci-après ou, éventuellement, pour l'injection d'un médicament liquide. Cette tige 24 pourra également comporter intérieurement une âme axiale métallique 52 la rigidifiant quelque peu telle un tube allongé délimitant le passage 50.

En relation avec les figures 1 à 7, on va maintenant décrire l'implantation du filtre 1 un peu en dessous des reins.

L'opérateur ménage d'abord dans la cuisse une voie d'accès percutanée de la veine fémorale interne gauche 4. Il introduit alors le fil 10, via l'orifice 7, dans la veine 4 jusqu'au vaisseau sanguin 2, un peu au-delà de la zone d'implantation 6.

Après l'élargissement de l'orifice 7, l'opérateur emmanche sur l'extrémité 28 de ce fil qui ressort de la veine fémorale, l'ensemble constitué du mandrin 20 introduit dans la gaine 8 (dont la longueur L_{g} est alors bien entendu au moins égale à la distance entre les zones 7 et 6).

Il fait ensuite descendre cet ensemble jusqu'à ce que le repère 34 du mandrin atteigne la zone 6. Le fil et le mandrin sont alors retirés, la gaine étant en position, son extrémité distale 82 arrivant dans la zone 6.

Après vissage sur l'embout 38 du corps 42 renfermant le filtre 1, l'opérateur fait alors glisser, via le poussoir 24, le filtre (toujours radialement contraint), jusqu'à l'expulser dans la zone d'implantation 6.

Avec une telle procédure, on constate dans certains cas une ouverture "dissymétrique" du filtre dont les appendices 14a, 14b et 14c (les plus proches de la paroi lors de l'éjection) apparaissent relativement groupés par rapport aux appendices 14d, 14e, 14f (figure 1).

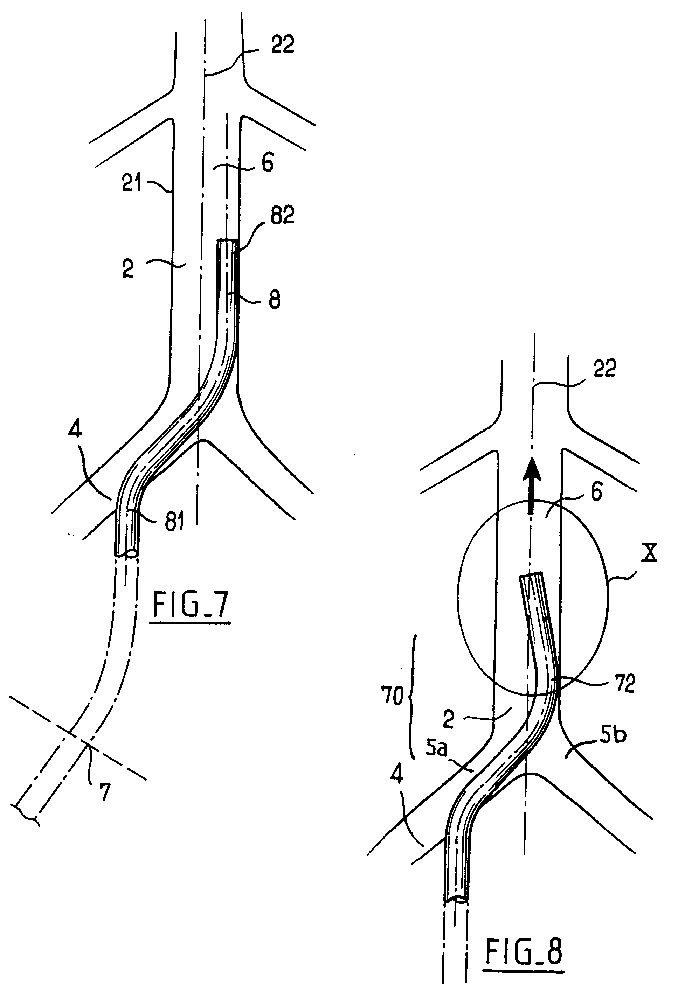

Etant parti du postulat que l'ouverture incorrecte du filtre pouvait être due à un problème de positionnement de la gaine, les inventeurs se sont attachés à modéliser ce positionnement. Ils ont ainsi constaté que la gaine pouvait se plaquer naturellement contre la paroi 21 de la veine 2, avec son axe 81 décentré par rapport à l'axe 22 (figure 7).

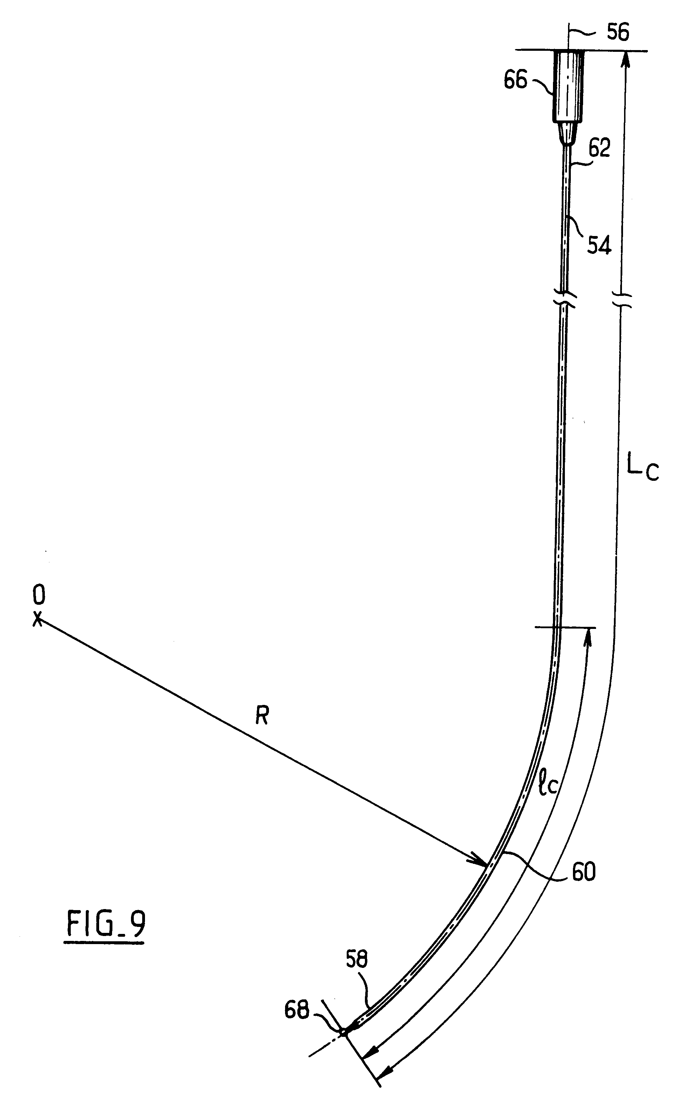

Pour résoudre cet inconvenient, l'invention propose d'utiliser des moyens de centrage 54 qui vont agir sur le profil de la ligne axiale de la gaine implantée, en astreignant son extrémité distale 82 à se diriger vers l'axe de la veine. Avantageusement, ces moyens seront montés glissants à l'intérieur de la gaine pour exercer donc une force sur la gaine tendant à modifier l'orientation de son extrémité 82. Tels qu'illustrés à la figure 8, ces moyens vont ainsi permettre, en fonction de leur position axiale dans la gaine et de leur position angulaire, de faire varier la courbure de cette dernière suivant son axe 81.

D'après la figure 9, ces moyens de centrage ou "de contrainte" pourront consister en un câble 54 relativement fin et flexible, ayant de préférence naturellement une rigidité supérieure ou égale à celle de la gaine et/ou de la tige 24.

De préférence, le câble sera également insérable dans la tige 24 creuse et présentera un diamètre extérieur légèrement inférieur au diamètre du passage 50 pour qu'il ne puisse y glisser qu'étroitement et sous contrainte.

On pourra faire subir au câble un traitement classique, de façon à lui assurer une forme naturellement courbée a priori différente de celle de la gaine (normalement déjà en position dans le vaisseau) et de toute façon telle que le câble ne puisse être disposé dans l'étroit passage 85 de cette gaine que dans un état contraint.

Le câble d'axe 56 pourra en outre comporter, à son extrémité distale 58, un bout 68 arrondi et être terminé, à son extrémité proximale 62, par une poignée élargie 66.

La longueur d'introduction du câble dans la tige et/ou la gaine sera a priori inférieure à la longueur de la gaine, puisqu'il est préférable que ce câble ne ressorte pas de son extrémité distale 82.

Toujours à la figure 9, ce fil de centrage 54, figuré ici dans son état naturel non contraint, présente à partir de son extrémité 62, une partie sensiblement rectiligne se poursuivant, vers l'extrémité 58 et sur une longueur l_{c}, par une partie terminale 60 de rayon de courbure R sensiblement constant. La longueur l_{c} sera de préférence comprise entre environ un quart et deux tiers de la longueur L_{c}. Le câble 54, élastiquement déformable suivant sa ligne axiale 56, présente avantageusement une flexibilité sensiblement constante ainsi qu'une constitution (par exemple en acier inoxydable) identique sur toute sa longueur L_{c}.

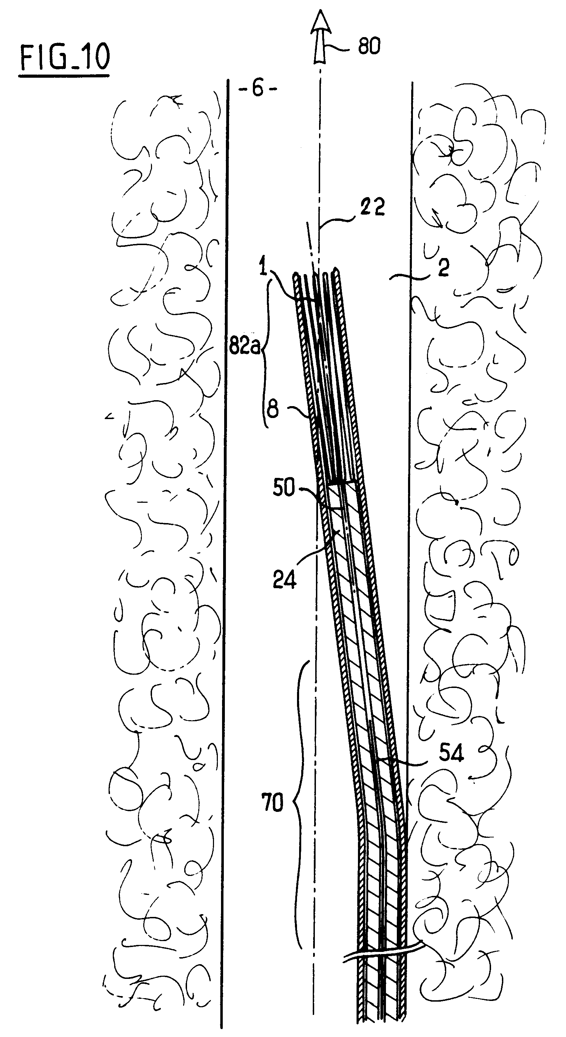

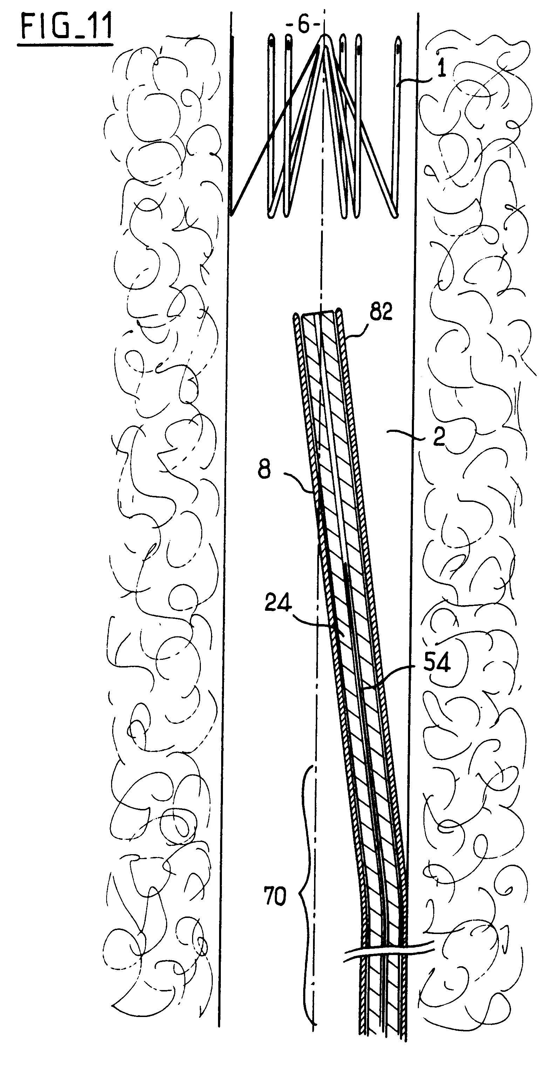

En se reportant aux figures 10 et 11, nous allons décrire une utilisation possible d'un tel fil 54.

Après avoir mis en place la gaine 8, la tige 24 pousse le filtre 1 dans la gaine en direction de son extrémité 82, mais en l'arrêtant au niveau de cette extrémité et sans le faire sortir de la gaine.

Le câble 54 est ensuite introduit, via l'embout creux 46, dans le passage 50 de la tige, jusque dans une zone 70 intérieure de la gaine située juste avant sa partie terminale distale 82a alors porteuse du filtre, légèrement en amont de la zone 6 et sensiblement à l'endroit du second méandre 72 que fait cette gaine, et au niveau ou juste après le raccordement des veines iliaques 5a et 5b (figure 8). C'est d'ailleurs sensiblement à partir de cette zone 70 que le dispositif est plaqué contre la paroi du vaisseau, avant l'introduction du câble 54. En jouant alors sur l'insertion plus ou moins profonde du câble dans la zone 70, il va être possible d'agir sur l'orientation axiale de l'extrémité 82, libre de mouvement, de la gaine en la dirigeant vers l'axe 22, à distance, depuis la zone 70, (l'effet produit tenant compte en particulier de la réaction de la gaine et de la paroi des vaisseaux contre lesquels ladite gaine porte, au moins localement).

Lorsque l'opérateur juge que l'extrémité 82 de la gaine est correctement positionnée et centrée dans la veine, il bloque alors le mouvement du câble et expulse le filtre en tirant légèrement en arrière la gaine. Le filtre doit alors correctement se positionner sensiblement axialement dans le vaisseau.

L'ensemble constitué par la gaine, la tige et le câble est retiré et l'incision pratiquée est refermée.

A titre indicatif, la longueur de la gaine pourra être de l'ordre de 60 à 70 cm, le fil 10, le mandrin et la tige 24 étant de préférence légèrement plus longs. La longueur L_{c} du câble pourra être comprise entre environ 70 et 90 cm, avec un diamètre de l'ordre de 1 mm, un rayon de courbure R d'environ 20 à 60 mm et une partie terminale 60 courbée définissant un angle compris entre environ 90° et 180°. Les diamètres extérieurs de la gaine, d'une part, du mandrin et de la tige 24, d'autre part, pourront être respectivement de l'ordre de 4 mm et 3 mm.

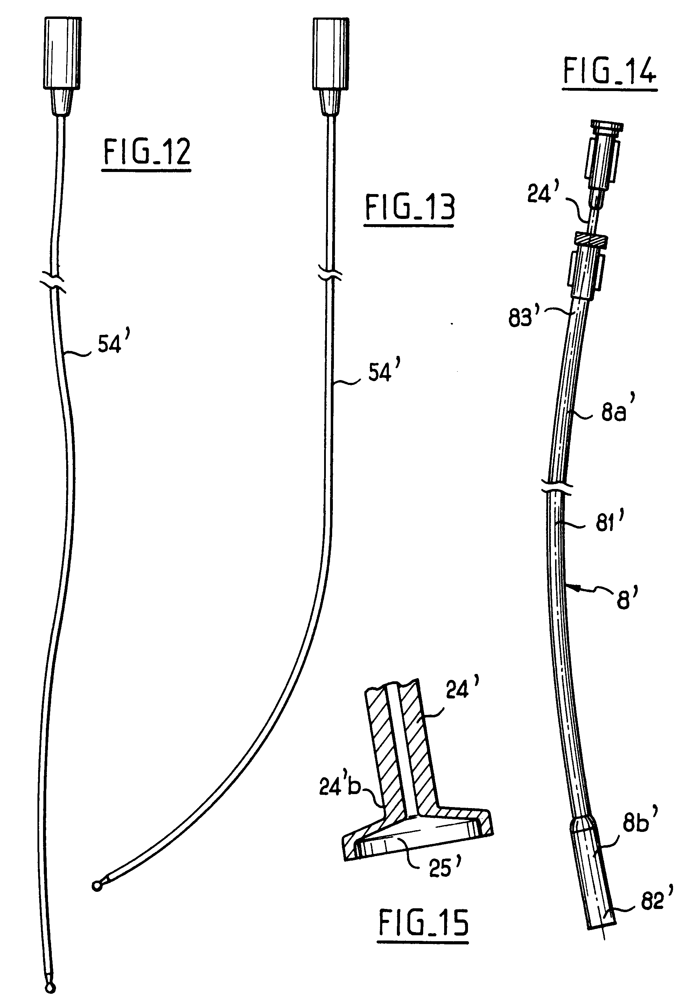

En variante, les moyens de centrage pourraient consister en un câble 54' (figures 12 et 13) réalisable en des matières à mémoire de forme, telles que des alliages à mémoire de forme thermique. Les températures auxquelles se produisent leur changement d'état dépendent notamment de la nature des alliages. Par exemple, pour des alliages comprenant environ 50% de titane et environ 50% de nickel, (tels du "Nitinol", marque déposée), la température de transition est voisine de celle du corps humain (habituellement d'environ 36°C à 38°C). Ainsi, le câble 54', traité de manière connue, pourra être souple et sans forme particulière (figure 12), à une température proche de celle ambiante (aux environs de 25°C) et pourra tendre à adopter naturellement (en conservant une certaine élasticité) une forme courbée un peu plus rigide à la température du corps du patient. Au contact de la gaine (et de la tige creuse) maintenue(s) en température par le sang du patient, un tel câble va donc reprendre sa courbure définie lors de son traitement, permettant ainsi à l'opérateur d'orienter à distance l'extrémité 82 de la gaine.

Le câble peut également être réalisé en un matériau à mémoire de forme pour présenter des propriétés d'élasticité très importantes. Ainsi les alliages précités du type "Nitinol" présentent dans leur phase élastique (structure austénitique) un taux d'allongement élastique compris entre environ 5 et 10 % et de préférence 6 et 8 % ; ce taux correspondant à la formule (dₘₐₓ-d) x 100/d, d étant la longueur nominale hors tension axiale d'une éprouvette réalisée en un tel alliage, dₘₐₓ étant la longueur maximale de cette même éprouvette sous contrainte axiale jusqu'à la limite de la zone d'élasticité, le résultat étant exprimé en pourcent (%).

Suivant une autre variante, la gaine 8 pourrait comporter un tube souple dont au moins une partie tubulaire, située dans la zone distale de la gaine, serait réalisée en un matériau à mémoire de forme, pour constituer lesdits moyens de centrage. Ainsi, lors de l'introduction de la gaine, la partie tubulaire serait progressivement chauffée par le sang jusqu'à la température de transition du matériau, reprenant alors sa forme courbée de centrage de la gaine dont la position de l'extrémité 82 pourrait être corrigée, si necessaire, par exemple grâce à la tige de commande introduite dans la gaine ou à un câble métallique.

Suivant encore une autre variante illustrée figure 14, on pourrait utiliser un dispositif d'implantation dit de Mobin-Uddin qui peut être mis en place par la technique de Seldinger (voir par exemple US-A-4727873). Un tel dispositif comprend une gaine tubulaire 8' qui comporte une partie principale souple 8a' , d'axe 81', prolongée par une partie terminale distale 8b' rigide, de plus fort diamètre, par exemple métallique. Cette partie 8b', beaucoup plus courte que la partie 8a', est adaptée pour recevoir le filtre rétracté avant l'introduction de la gaine dans le corps du patient. Quant à la tige 24', elle est prolongée par une partie distale évasée creuse 25', de diamètre supérieur à celui de la tige mais bien entendu légèrement inférieur au diamètre intérieur de la partie terminale de la gaine, formant une surface d'appui pour expulser le filtre hors de la gaine.

## Revendications

1. Dispositif d'implantation d'une prothèse médicale (1) dans un conduit (2) d'un corps humain ou animal, ledit conduit présentant un axe (22), ce dispositif comprenant :
- une gaine tubulaire (8, 8') d'implantation adaptée pour pouvoir contenir au moins localement ladite prothèse à implanter, ladite gaine, qui est allongée suivant une ligne axiale (81, 81'), présentant une extrémité distale (82, 82') et étant flexible sur une partie au moins de sa longueur,
- une tige (24, 24') de commande de la prothèse, ladite tige étant au moins en partie flexible et de diamètre extérieur adapté pour glisser dans la gaine,
- et des moyens de centrage (54, 54') propres à agir sur le profil de la ligne axiale de la gaine, caractérisé en ce que ces moyens de centrage, flexibles, sont insérés axialement de manière glissante à l'intérieur de la tige de commande (24, 24') ou la gaine (8, 8'), sur une partie au moins de leur longueur, pour exercer une force interne sur ladite partie flexible de la gaine, en tendant par réaction, en fonction de leur insertion plus ou moins profonde dans la tige et/ou la gaine, à faire varier l'orientation de l'extrémité distale (82, 82') de cette gaine.

2. Dispositif selon la revendication 1, caractérisé en ce que lesdits moyens de centrage comprennent un câble flexible (54, 54') présentant une ligne axiale (56) courbée pour agir sur le profil de la ligne axiale de la gaine.

3. Dispositif selon la revendication 1 ou la revendication 2, caractérisé en ce que la tige de commande (24, 24') est un tube creux au moins en partie, propre à recevoir lesdits moyens de centrage.

4. Dispositif selon l'une quelconque des revendications 1 à 3, caractérisé en ce que la longueur d'introduction desdits moyens de centrage (54, 54') à l'intérieur de la tige de commande ou de la gaine est inférieure à la longueur (L_{g}) de cette dernière, pour qu'ils contraignent à distance son extrémité distale (82, 82').

5. Dispositif selon l'une quelconque des revendications 3 ou 4, caractérisé en ce que le câble présente un diamètre extérieur au moins légèrement inférieur au diamètre du passage intérieur (50) de la tige de commande (24, 24'), pour y glisser étroitement, sous contrainte.

6. Dispositif selon l'une quelconque des revendications 2 à 5, caractérisé en ce que le câble est réalisé en un matériau à mémoire de forme thermique.

7. Dispositif selon l'une quelconque des revendications 2 à 6, caractérisé en ce que le câble est réalisé en un alliage comprenant environ 50% de titane et 50% de nickel.

8. Dispositif selon l'une quelconque des revendications 2 à 7, caractérisé en ce que le taux d'allongement élastique axial du câble est compris entre environ 5% et 10% et de préférence 6% et 8%.

9. Dispositif selon l'une quelconque des revendications précédentes, caractérisé en ce que la gaine (8) est souple sur toute sa longueur, étant dépourvue de partie terminale distale rigide propre à contenir la prothèse.

10. Application du dispositif selon l'une quelconque de revendications 1 à 9, dans laquelle ledit conduit est un vaisseau sanguin (2) et ladite prothèse est un filtre sanguin définitif (1) équipé de moyens d'accrochage (16) à la paroi du vaisseau et expansible radialement, pour la retenue d'éventuels caillots de sang.
